# EUROPEAN PATENT APPLICATION

(11) **EP 3 323 469 A1**
(43) Date of publication of application: **23.05.2018**
(21) Application number: 16425108.4
(22) Date of filing: 21.11.2016
(51) Int. Cl.: A61Q 3/02, A61K 8/19, A61K 8/26, A61K 8/29, A61K 8/35, A61K 8/46, A61K 8/49, A45D 44/00, B44D 3/00

(54) **COLOURING COMPOSITIONS**

(71) Applicant: J Colors S.p.A., 20020 Lainate, Milano (IT)
(72) Inventor: Junghanns, Carlo Alberto Goebel, I-20020 Lainate, Milano (IT)
(74) Representative: Carangelo, Pierluigi

(57) **Abstract**

The present invention relates to a set of dye compositions, particularly suitable for preparing lacquers.

## Description

The present invention relates to a set of dye compositions, particularly adapted for preparing nail lacquers.

### Technical field of the invention

In the cosmetics and make-up industry, including nail make-up, fashion and trends dictate an increasingly changing range of colors, nuances and shades.

Therefore, being prepared to put on the market products that meet the expectations of customers is crucial.

The preparation of colored compositions is obtained by mixing different colors; in order to prepare specific nuances, it may be necessary to mix three or more colors with one other.

This results in the need to have a wide range of colored preparations available, so that they can be taken when necessary.

Considering that there are about 2,300 colors available, it is clear that the supply and storage of such a number of colors is not always economically feasible and can still be very cumbersome and certainly expensive.

### Summary of the invention

The object of the present invention is therefore to provide a set of a discrete number of compositions that may be used for the preparation of a very wide range of colored compositions for nail lacquers.

### Object of the invention

A first object of the present invention is to provide a set (or sets) of dye compositions and, in particular objects of the invention, subsets thereof.

According to a second object thereof, a method is described for preparing a colored composition starting from the set of dye compositions of the invention.

Last but not least, the present invention describes the use of thirteen dye compositions plus two basic compositions for preparing a colored composition having any predetermined and desired color.

### Detailed description of the invention

### Definitions

The term "dye composition" means any of the compositions forming the set of compositions described in the present description.

The term "set of dye compositions" (or briefly, the "set") means the group of a discrete number of dye compositions provided by the present invention.

The term "subset of dye compositions" (or briefly, the "subset") means a group comprising one or more of the dye compositions making up the set of the invention.

The term "colored composition" means any composition having a specific predetermined color which may be prepared using the set or subset of dye compositions of the present invention.

The term "lacquer" means a composition suitable and acceptable from the cosmetic point of view, for use in nail make-up.

According to the first object of the invention, a set of dye compositions is described.

According to a preferred embodiment, the set of dye compositions consists of:
- a Fundamental Composition (FC),
- Basic Dye Compositions (BC), and
- Elementary Dye Compositions (EC).

For the purposes of the present invention, the Fundamental Composition (FC) may also be a component of any of the Basic Dye Compositions (BC) and/or of any of the Elementary Dye Compositions (EC).

According to a preferred embodiment, the Fundamental Composition (FC) is a component of any of the Basic Dye Compositions (BC) and of any of the Elementary Dye Compositions (EC).

The Fundamental Composition (FC) is a mixture of components and, in a preferred embodiment, has the following composition:

| **COMPONENT** | **part/1000 parts** |
|---|---|
| Modified polyester resin 75% solids in ethyl acetate | **5 - 15** |
| Solvent Acetate | **550 - 650** |
| Thermoplastic Acrylic resin | **20 - 30** |
| Hectorite | **10 - 15** |
| Modified Montmorillonite | **2 - 7** |
| Tosylamide resin 74% solids in butyl acetate | **75 - 95** |
| Silicon dioxide | **4 - 6** |
| Nitrocellulose | **170 - 190** |
| Plasticizer | **55 - 65** |
| Soya lecithin | **2.5 - 4** |
| Antioxidant | **26 - 30** |
| Perfumes | **2.5 - 3.5** |
| TOTAL | **1000** |

As said above, the Fundamental Composition (FC) may be, and preferably is, a component of the Basic Dye Compositions (BC).

In a preferred embodiment, the Fundamental Composition (FC) represents about 0.5-55% and more preferably about 1-5% (weight/weight) of the Basic Dye Composition (BC).

With regard to each of the Basic Dye Compositions (BC1 and BC2), in one aspect of the present invention these are two in number.

More in detail, each of the Basic Compositions includes a basic color.

In particular, the first of the two Basic Dye Compositions described above (BC1) comprises a basic color which is preferably represented by black.

In a preferred embodiment, the basic color BC1 is Carbon Black.

The second of the two Basic Dye Compositions described above (BC2) comprises a basic color which is preferably represented by white.

In a preferred embodiment, the basic color BC2 is titanium dioxide (77981 color index).

### Elementary Dye Compositions (EC)

According to the present invention, each Elementary Dye Composition (EC) comprises a basic color and the Fundamental Composition (FC).

The Fundamental Composition of the EC, in particular, corresponds to that described above.

The basic colors of the Elementary Compositions (EC), instead, are different from those of the BC1 and BC2 and are described hereinafter.

In particular, Elementary Dye Compositions (EC) comprising the following basic colors are described:

| |
|---|
| 77742 (color index) |
| 77288 (color index) |
| 77289 (color index) |
| 47005 (color index) |
| 15850 (color index) CAS no. 5858-81-1 |
| 77510 (color index) |
| 15880 (color index) |
| 77492 (color index) |
| 19140 (color index) |
| 15850 (color index) CAS no. 5281-04-9 |
| 15850 (color index) CAS no. 17852-98-1 |
| 77007 (color index) |
| 42090 (color index) |

According to the present invention, therefore, each of the: Fundamental Composition (FC), Basic Dye Compositions (BC) and Elementary Dye Compositions (EC) is an object of the present invention.

Moreover, any subset of two or more elements, in which each element is selected independently of one another from the Fundamental Composition (FC), each of the Basic Dye Compositions (BC) and the Elementary Dye Compositions (EC) described above, is a further object of the invention.

The set consisting of all thirteen Elementary Dye Compositions (EC) and the two Basic Compositions (BC) is a preferred object of the invention.

In this case, the Fundamental Composition (FC) is not part of the set as a separate composition, but it is clear that it may be a portion of the chemical composition of any one of the Basic Dye Compositions (CB1 and CB2) and of the Elementary Dye Compositions.

The set consisting of all thirteen Elementary Dye Compositions (EC), the two Basic Compositions (BC) and the Fundamental Composition (FC) is another preferred object of the invention.

According to a second object of the present invention, a method is described for preparing a Colored Composition starting from any combination of the Dye Compositions described above.

In particular, said method comprises the steps of:
1) providing a set of Dye Compositions;
2) mixing a suitable amount of one or more of the Dye Compositions of said set to obtain a predetermined and desired color;
wherein the set of Dye Compositions of step 1) consists of sixteen Dye Compositions.

According to a preferred embodiment of the invention, such sixteen Dye Compositions consist of:
- Fundamental Composition (FC),
- Basic Dye Compositions (BC), and
- Elementary Dye Compositions (EC),
are those described above.

According to a preferred embodiment, the set of Dye Compositions of step 1) consists of fifteen dye compositions.

In particular, said fifteen compositions comprise the two Basic Dye Compositions (BC1 and BC2) and thirteen Elementary Dye Compositions; the Fundamental Composition (FC) is excluded as a separate composition (but may be a constituent of each of the Basic Compositions or Elementary Compositions).

In particular, in step 2), a suitable amount should be understood as an amount necessary to obtain the specific, predetermined and desired final color.

It is noted that for the purposes of the present invention, the dye compositions are preferably provided with methods that exclude the direct sale to the consumer.

Such methods relate in particular to the amount of each of the Dye Compositions and to the packaging.

For example, each Composition is preferably provided in containers (cans, jars, bins) with typical volumes of wholesale rather than retail.

It is therefore excluded that the direct consumer, typically represented by the purchaser of nail lacquers, may take advantage of the present invention which is instead preferably directed to nail lacquer manufacturers and successively placed on the market by them.

In a mostly preferred embodiment, the colored composition prepared by the above method is a cosmetic composition.

According to a preferred embodiment, such a cosmetic composition is represented by the nail make-up lacquer.

In the following examples, the amounts are expressed as parts per 1000 parts by weight.

### EXAMPLE 1

### Fundamental Composition (FC)

| **COMPONENT** | **part/1000 parts** |
|---|---|
| Modified polyester resin 75% solids in ethyl acetate | **5 - 15** |
| Solvent Acetate | **550 - 650** |
| Thermoplastic Acrylic resin | **20 - 30** |
| Hectorite | **10 - 15** |
| Modified Montmorillonite | **2 - 7** |
| Tosylamide resin 74% solids in butyl acetate | **75 - 95** |
| Silicon dioxide | **4 - 6** |
| Nitrocellulose | **170 - 190** |
| Plasticizer | **55 - 65** |
| Soya lecithin | **2.5 - 4** |
| Antioxidant | **26 - 30** |
| Perfumes | **2.5 - 3.5** |
| TOTAL | **1000** |

### EXAMPLES 2-3

### Basic Dye Compositions

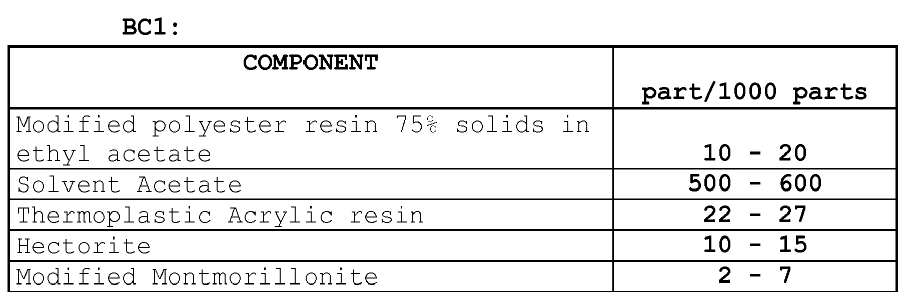

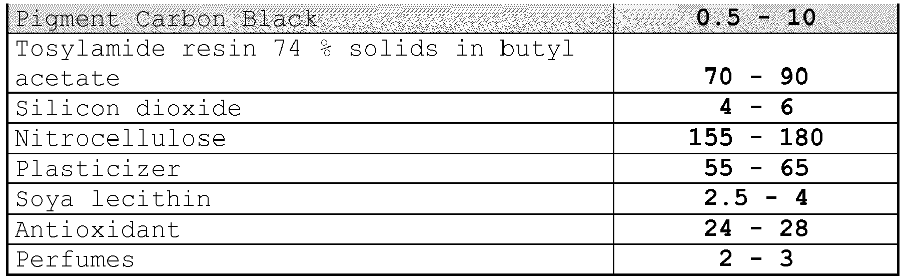

**BC2:**

| **COMPONENT** | **part/1000 parts** |
|---|---|
| Modified polyester resin 75% solids in ethyl acetate | **20 - 50** |
| Solvent Acetate | **500 - 600** |
| Thermoplastic Acrylic resin | **22 - 27** |
| Bentonite | **0.5 - 1.5** |
| Hectorite | **10 - 15** |
| Modified Montmorillonite | **2 - 7** |
| Pigments Titanium Dioxide 77891 color index | **20 - 30** |
| Tosylamide resin 74 % solids in butyl acetate | **70 - 90** |
| Silicon dioxide | **4** - **6** |
| Nitrocellulose | **155 - 180** |
| Plasticizer | **50 - 60** |
| Soya lecithin | **2.5 - 4** |
| Antioxidant | **24 - 28** |
| Perfumes | **2** - **3** |

### EXAMPLES 4-16

### Elementary Dye Compositions (EC)

**EC1:**

| **COMPONENT** | **part/1000 parts** |
|---|---|
| Modified polyester resin 75% solids in ethyl acetate | **50 - 80** |
| Solvent Acetate | **500 - 600** |
| Thermoplastic Acrylic resin | **25 - 30** |
| Bentonite | **0.1 - 1** |
| Hectorite | **10 - 15** |
| Modified Montmorillonite | **2 - 7** |
| Pigments 15850 color index CAS no. 5858-81-1 | **10 - 20** |
| Tosylamide resin 74 % solids in butyl acetate | **70 - 90** |
| Silicon dioxide | **4 - 6** |
| Nitrocellulose | **155 - 180** |
| Plasticizer | **50 - 60** |
| Soya lecithin | **2.5 - 4** |
| Antioxidant | **24 - 28** |
| Perfumes | **2 - 3** |

**EC2:**

| **COMPONENT** | **part/1000 parts** |
|---|---|
| Modified polyester resin 75% solids in ethyl acetate | **35 - 65** |
| Solvent Acetate | **500 - 600** |
| Thermoplastic Acrylic resin | **20 - 25** |
| Bentonite | **0.1 - 1** |
| Hectorite | **10 - 15** |
| Modified Montmorillonite | **2 - 7** |
| Pigment 15850 color index CAS No. 5281-04-9 | **5 - 15** |
| Tosylamide resin 74 % solids in butyl acetate | **70 - 90** |
| Silicon dioxide | **4 - 6** |
| Nitrocellulose | **155 - 180** |
| Plasticizer | **50 - 60** |
| Soya lecithin | **2.5 - 4** |
| Antioxidant | **24 - 28** |
| Perfumes | **2 - 3** |

**EC3:**

| **COMPONENT** | **part/1000 parts** |
|---|---|
| Modified polyester resin 75% solids in ethyl acetate | **35 - 65** |
| Solvent Acetate | **500 - 600** |
| Thermoplastic Acrylic resin | **20 - 25** |
| Bentonite | **0.1 - 1** |
| Hectorite | **10 - 15** |
| Modified Montmorillonite | **2 - 7** |
| Pigment 15850 color index CAS no. 17852-98-1 | **10 - 20** |
| Tosylamide resin 74 % solids in butyl acetate | **70 - 90** |
| Silicon dioxide | **4 - 6** |
| Nitrocellulose | **155 - 180** |
| Plasticizer | **50 - 60** |
| Soya lecithin | **2.5 - 4** |
| Antioxidant | **24 - 28** |
| Perfumes | **2 - 3** |

**EC4:**

| **COMPONENT** | **part/1000 parts** |
|---|---|
| Modified polyester resin 75% solids in ethyl acetate | **35 - 65** |
| Solvent Acetate | **500 - 600** |
| Thermoplastic Acrylic resin | **25 - 30** |
| Bentonite | **0.5 - 1.5** |
| Hectorite | **10 - 15** |
| Modified Montmorillonite | **2 - 7** |
| Pigments 19140 color index | **10 - 20** |
| Tosylamide resin 74 % solids in butyl acetate | **70 - 90** |
| Silicon dioxide | **4** - **6** |
| Nitrocellulose | **155 - 180** |
| Plasticizer | **50 - 60** |
| Soya lecithin | **2.5 - 4** |
| Antioxidant | **24 - 28** |
| Perfumes | **2** - **3** |

**EC5:**

| **COMPONENT** | **part/1000 parts** |
|---|---|
| Modified polyester resin 75% solids in ethyl acetate | **50 - 80** |
| Solvent Acetate | **500 - 600** |
| Thermoplastic Acrylic resin | **25 - 30** |
| Bentonite | **1 - 2** |
| Hectorite | **10 - 15** |
| Modified Montmorillonite | **2 - 7** |
| Pigments 47005 color index | **15 - 25** |
| Tosylamide resin 74 % solids in butyl acetate | **70 - 90** |
| Silicon dioxide | **4 - 6** |
| Nitrocellulose | **155 - 180** |
| Plasticizer | **50 - 60** |
| Soya lecithin | **2.5 - 4** |
| Antioxidant | **24 - 28** |
| Perfumes | **2** - **3** |

**EC6:**

| **COMPONENT** | **part/1000 parts** |
|---|---|
| Modified polyester resin 75% solids in ethyl acetate | **20 - 50** |
| Solvent Acetate | **500 - 600** |
| Thermoplastic Acrylic resin | **25 - 30** |
| Bentonite | **1.5 - 2.5** |
| Hectorite | **10 - 15** |
| Modified Montmorillonite | **2 - 7** |
| Pigments 77289 color index | **40 - 50** |
| Tosylamide resin 74 % solids in butyl acetate | **70 - 90** |
| Silicon dioxide | **4 - 6** |
| Nitrocellulose | **155 - 180** |
| Plasticizer | **50 - 60** |
| Soya lecithin | **2.5 - 4** |
| Antioxidant | **24 - 28** |
| Perfumes | **2 - 3** |

**EC7:**

| **COMPONENT** | **part/1000 parts** |
|---|---|
| Modified polyester resin 75% solids in ethyl acetate | **20 - 50** |
| Solvent Acetate | **500 - 600** |
| Thermoplastic Acrylic resin | **25 - 30** |
| Bentonite | **1 - 2** |
| Hectorite | **10 - 15** |
| Modified Montmorillonite | **2 - 7** |
| Pigments 77007 color index | **40 - 50** |
| Tosylamide resin 74 % solids in butyl acetate | **70 - 90** |
| Silicon dioxide | **4 - 6** |
| Nitrocellulose | **155 - 180** |
| Plasticizer | **50 - 60** |
| Soya lecithin | **2.5 - 4** |
| Antioxidant | **24 - 28** |
| Perfumes | **2 - 3** |

**EC8:**

| **COMPONENT** | **part/1000 parts** |
|---|---|
| Modified polyester resin 75% solids in ethyl acetate | **20 - 50** |
| Solvent Acetate | **500 - 600** |
| Thermoplastic Acrylic resin | **25 - 30** |
| Bentonite | **1.5 - 2.5** |
| Hectorite | **10 - 15** |
| Modified Montmorillonite | **2 - 7** |
| Pigments 77742 color index | **40 - 50** |
| Tosylamide resin 74 % solids in butyl acetate | **70 - 90** |
| Silicon dioxide | **4 - 6** |
| Nitrocellulose | **155 - 180** |
| Plasticizer | **50 - 60** |
| Soya lecithin | **2.5 - 4** |
| Antioxidant | **24 - 28** |
| Perfumes | **2 - 3** |

**EC9:**

| **COMPONENT** | **part/1000 parts** |
|---|---|
| Modified polyester resin 75% solids in ethyl acetate | **20 - 50** |
| Solvent Acetate | **500 - 600** |
| Thermoplastic Acrylic resin | **25 - 30** |
| Bentonite | **0.5 - 1.5** |
| Hectorite | **10 - 15** |
| Modified Montmorillonite | **2 - 7** |
| Pigments 77492 color index | **15 - 25** |
| Tosylamide resin 74 % solids in butyl acetate | **70 - 90** |
| Silicon dioxide | **4 - 6** |
| Nitrocellulose | **155 - 180** |
| Plasticizer | **50 - 60** |
| Soya lecithin | **2.5 - 4** |
| Antioxidant | **24 - 28** |
| Perfumes | **2 - 3** |

**EC10:**

| **COMPONENT** | **part/1000 parts** |
|---|---|
| Modified polyester resin 75% solids in ethyl acetate | **35 - 65** |
| Solvent Acetate | **500 - 600** |
| Thermoplastic Acrylic resin | **25 - 30** |
| Bentonite | **0.5 - 1.5** |
| Hectorite | **10 - 15** |
| Modified Montmorillonite | **2 - 7** |
| Pigments 77510 color index | **5 - 15** |
| Tosylamide resin 74 % solids in butyl acetate | **70 - 90** |
| Silicon dioxide | **4 - 6** |
| Nitrocellulose | **155 - 180** |
| Plasticizer | **50 - 60** |
| Soya lecithin | **2.5 - 4** |
| Antioxidant | **24 - 28** |
| Perfumes | **2 - 3** |

**EC11:**

| **COMPONENT** | **part/1000 parts** |
|---|---|
| Modified polyester resin 75% solids in ethyl acetate | **20 - 50** |
| Solvent Acetate | **500 - 600** |
| Thermoplastic Acrylic resin | **25 - 30** |
| Bentonite | **1.5 - 2.5** |
| Hectorite | **10 - 15** |
| Modified Montmorillonite | **2 - 7** |
| Pigments 77288 color index | **35 - 45** |
| Tosylamide resin 74 % solids in butyl acetate | **70 - 90** |
| Silicon dioxide | **4 - 6** |
| Nitrocellulose | **155 - 180** |
| Plasticizer | **50 - 60** |
| Soya lecithin | **2.5 - 4** |
| Antioxidant | **24 - 28** |
| Perfumes | **2** - **3** |

**EC12:**

| **COMPONENT** | **part/1000 parts** |
|---|---|
| Modified polyester resin 75% solids in ethyl acetate | **35 - 65** |
| Solvent Acetate | **500 - 600** |
| Thermoplastic Acrylic resin | **22 - 27** |
| Bentonite | **0.1 - 1** |
| Hectorite | **10 - 15** |
| Modified Montmorillonite | **2 - 7** |
| Pigments 42090 color index | **5 - 15** |
| Tosylamide resin 74 % solids in butyl | **70 - 90** |
| acetate | |
| Silicon dioxide | **4 - 6** |
| Nitrocellulose | **155 - 180** |
| Plasticizer | **50 - 60** |
| Soya lecithin | **2.5 - 4** |
| Antioxidant | **24 - 28** |
| Perfumes | **2 - 3** |

**EC13:**

| **COMPONENT** | **part/1000 parts** |
|---|---|
| Modified polyester resin 75% solids in ethyl acetate | **50 - 80** |
| Solvent Acetate | **500 - 600** |
| Thermoplastic Acrylic resin | **22 - 27** |
| Bentonite | **0.5 - 1.5** |
| Hectorite | **10 - 15** |
| Modified Montmorillonite | **2 - 7** |
| Pigments 15880 color index | **10 - 20** |
| Tosylamide resin 74 % solids in butyl acetate | **70 - 90** |
| Silicon dioxide | **4 - 6** |
| Nitrocellulose | **155 - 180** |
| Plasticizer | **50 - 60** |
| Soya lecithin | **2.5 - 4** |
| Antioxidant | **24 - 28** |
| Perfumes | **2 - 3** |

According to a further object, the present invention describes the use of a set of Dye Compositions for preparing a (final) colored composition having any specific, predetermined and desired color.

According to one aspect of the present invention, such a set of dye compositions comprises:
- two Basic Dye Compositions, and
- thirteen Elementary Dye Compositions.

The Basic Dye Compositions and the Elementary Dye Compositions are those described above.

In one aspect of the invention, such a set further comprises a Fundamental Composition (FC) which, for the purposes of the present invention, is the one described above.

The use described by the present invention, in particular, is for the preparation of a Colored Composition represented by a cosmetic preparation which, in a preferred aspect, is a nail lacquer.

The several advantages of the present invention are apparent from the above description.

It is first noted that the discrete set of Dye Compositions of the present invention is the minimum set of dye compositions, thus sufficient but necessary, to obtain any desired colored composition.

In particular, such a discrete set was carefully selected by the inventors among the more than 2,300 dye compositions available.

In fact, any addition of colors to such a set was found to be unnecessary.

The inventors have surprisingly found that the selected compositions forming the set are compatible and can thus be mixed in any amount without problems of stability, homogeneity, segregation, where stability also means the ability to maintain the color over time.

Therefore, it will be sufficient to have the set of the invention available to prepare the required final colors, without the need of having any color, hue or shade in stock, with a clear saving on costs, time, organization and storage space.

In particular, the discrete set of dye compositions of the invention allows to obtain a very large, almost infinite number of colors, hues, tones and shades.

Those skilled in the art may make changes or adaptations to the present invention, without, however, departing from the scope of the following claims.

## Claims

1. A set of Dye Compositions consisting of:
- Basic Dye Compositions (BC), and
- Elementary Dye Compositions (EC),
**characterized in that** the Basic Dye Compositions (BC) are two and that the Elementary Dye Compositions (EC) are thirteen.

2. A set of Dye Compositions according to the preceding claim, further comprising a Fundamental Composition, as such, which is separate from the Basic Dye Compositions (BC) and from the Elementary Dye Compositions.

3. A set of Dye Compositions according to claim 1, wherein the Basic Dye Compositions (BC) and the Elementary Dye Compositions (EC) have a composition which includes a portion of the Fundamental Composition (FC).

4. A set of Dye Compositions according to claim 2 or 3, wherein the Fundamental Composition (FC) has the following composition:
| Modified polyester resin 75% solids in ethyl acetate |
|---|
| Solvent Acetate |
| Thermoplastic Acrylic resin |
| Bentonite |
| Hectorite |
| Modified Montmorillonite |
| Tosylamide resin 74 % solids in butyl |
| acetate |
| Silicon dioxide |
| Nitrocellulose |
| Plasticizer |
| Soya lecithin |
| Antioxidant |
| Perfumes |

5. A set of Dye Compositions according to claim 1 or 2 or 3, wherein the Fundamental Composition (FC) has the following composition:
| **COMPONENT** | **part/1000 parts** |
|---|---|
| Modified polyester resin 75% solids in ethyl acetate | **5 - 15** |
| Solvent Acetate | **550 - 650** |
| Thermoplastic Acrylic resin | **20 - 30** |
| Hectorite | **10 - 15** |
| Modified Montmorillonite | **2 - 7** |
| Tosylamide resin 74% solids in butyl acetate | **75 - 95** |
| Silicon dioxide | **4 - 6** |
| Nitrocellulose | **170 - 190** |
| Plasticizer | **55 - 65** |
| Soya lecithin | **2.5 - 4** |
| Antioxidant | **26 - 30** |
| Perfumes | **2.5 - 3.5** |
| TOTAL | **1000** |

6. A set of Dye Compositions according to any one of the preceding claims, wherein one of the two Basic Compositions (BC) comprises Carbon Black as the basic color.

7. A set of Dye Compositions according to any one of the preceding claims, wherein one of the two Basic Compositions (BC) comprises titanium dioxide (77891 color index) as the basic color.

8. A set of Dye Compositions according to any one of the preceding claims, wherein each Dye Composition comprises about 0.5-55% and more preferably about 1-5% (weight/weight%) of the Fundamental Composition (FC).

9. A set of Dye Compositions according to any one of the preceding claims, wherein the Elementary Dye Compositions are **characterized by** comprising one of the following basic colors:
| |
|---|
| 77742 (color index) |
| 77288 (color index) |
| 77289 (color index) |
| 47005 (color index) |
| 15850 (color index) CAS no. 5858-81-1 |
| 77510 (color index) |
| 15880 (color index) |
| 77492 (color index) |
| 19140 (color index) |
| 15850 (color index) CAS no. 5281-04-9 |
| 15850 (color index) CAS no. 17852-98-1 |
| 77007 (color index) |
| 42090 (color index) |

10. A method for preparing a Colored Composition comprising the steps of:
1) providing a set of Dye Compositions;
2) mixing a suitable amount of two or more of the Dye Compositions of said set so as to obtain a Colored Composition having a predetermined color;
wherein the set of Dye Compositions of step 1) consists of:
- the two Basic Dye Compositions (BC) of any one of claims 1 or 6 or 7, and
- the thirteen Elementary Dye Compositions (EC) according to claim 4 or 5.

11. A method according to the preceding claim, wherein in step 2) a suitable amount is the amount required to obtain a specific final color.

12. A method according to any one of claims 10 to 12, wherein said Colored Composition is a nail make-up lacquer.

13. Use of a set of Dye Compositions according to any one of claims 1 to 9 for preparing a Colored Composition having a predetermined color.

14. Use according to the preceding claim, wherein said Colored Composition is a nail lacquer.
